# EUROPEAN PATENT APPLICATION

(11) **EP 1 548 437 A1**
(43) Date of publication of application: **29.06.2005**
(21) Application number: 03797637.0
(22) Date of filing: 17.09.2003
(51) Int. Cl.: G01N 33/53, G01N 33/543, G01N 37/00

(54) **METHOD AND APPARATUS FOR ARRANGING LIQUID REACTION COMPONENTS ON SUBSTRATE SURFACE FOR DETECTING TARGET SUBSTANCE BY REACTION AMONG PLURAL REACTION COMPONENTS ON SUBSTRATE AND ARTICLE UTILIZED IN THE METHOD**

(30) Priority: 17.09.2002 JP 2002270527
(71) Applicant: Olympus Corporation, Tokyo 151-0072 (JP)
(72) Inventor: HAMANO, Tamaki, Hidaka-shi, Saitama 350-1234 (JP)
(74) Representative: von Hellfeld, Axel, Dr. Dipl.-Phys.
(86) International application number: PCT/JP2003/011850
(87) International publication number: WO 2004/027422

(57) **Abstract**

Disclosed is a method of carrying out a specific coupling reaction between a first component and a second component collectively forming a specific coupling pair by supplying the second component onto a plurality of isolated reaction regions formed on the substrate surface and having the first component arranged therein in advance, comprising the step of regioselectively arranging a liquid material containing the second component in a manner to cover the reaction region having the first component arranged therein in advance in accordance with the address of each reaction region. The present invention also provides an apparatus and an article for carrying out the method.

## Description

### Technical Field

The present invention relates to the detection of a target substance in a large number of isolated reaction reactions on a substrate surface by utilizing a specific coupling reaction as in the assay method using micro-array. More particularly, the present invention relates to a method of dispensing the liquid components required for the specific coupling reaction onto the substrate surface. The present invention also relates to an apparatus for dispensing the liquid components on the substrate surface. Further, the present invention relates to an article useful for performing the method noted above.

### Background Art

The living body is controlled by the expression of various physiologically active substances present in the living body texture. A micro-array is used nowadays as the main means of the gene expression frequency analysis that controls the expression of the physiologically active substances. The micro-array is prepared by arranging and immobilizing probes such as DNA, RNA and proteins on a substrate such as a slide glass or a silicon substrate in a density of thousands of probes per square centimeter. For example, a micro-array prepared by immobilizing nucleic acid probes on a solid phase substrate is used as a means for analyzing the expression frequency of the target DNA and target RNA, because the immobilized probes are specifically coupled with target DNA or RNA by the hybridization reaction.

In forming the micro-array, the three technologies of photolithography (light immobilization), ink jetting and mechanical micro-spotting are mainly used for preparing the micro-array, in order to spotting trace amount of probes on the substrate surface. The hybridization reaction between the trace amount of probes immobilized on the substrate surface and a target contained in a sample solution is carried out in general as follows. Specifically, the micro-array having the probes immobilized thereon is covered with, for example, a cover glass and, then, a target solution is injected into the clearance between the cover glass and the micro-array so as to carry out the hybridization reaction between the probes and the target contained in the sample solution. The cover glass noted above serves to prevent the evaporation of the sample solution.

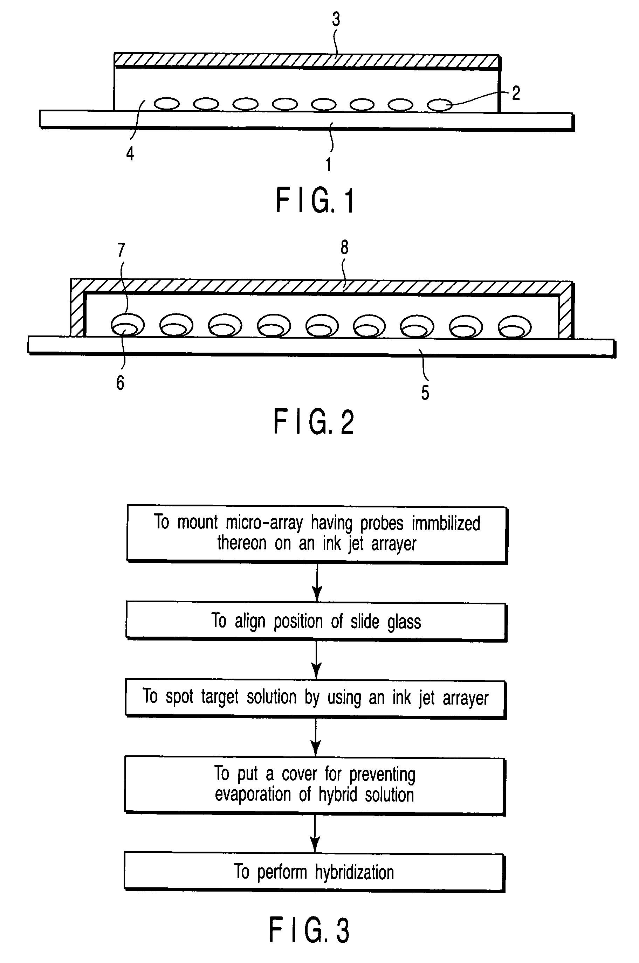

For example, in the hybridization reaction assays using an ordinary slide glass as shown in FIG. 1, a lid member such as a cover glass 3 is arranged so as to face probes 2 such as trace amount of DNA immobilized on a micro-array substrate 1 such as a slide glass, and the clearance between the cover glass 3 and the probes 2 is filled with a sample solution 4 containing a target, thereby forming a hybrid between the probe and the target.

For improving the efficiency of the hybridization, an improved method of the hybridization utilizing the electrical characteristics of DNA is also known to the art (see, for example, FIG. 1 of Japanese Patent Disclosure (Kokai) No. 8-154656).

However, during the hybridization reaction carried out by using the conventional array, only a small amount of the sample solution, which is present in the vicinity of the probes, is effectively utilized for the hybrid formation, and it is difficult to utilize the sample solution that is not present in the vicinity of the probes.

Also, a phenomenon of a high background noise is observed in the conventional array. The background noise noted above refers to the phenomenon that signals are detected in regions to which the probes have not been immobilized, depending on the amount of the fluorescent substance used as a marker and the composition of the solution used in the step of the hybridization. If the background noise is high, the detection sensitivity of the actual hybridization signal is lowered.

Further, where a cover glass for hybridization is used together with trace amount of probes that are immobilized on a single micro-array in the conventional method, it was necessary to use a large amount of the target solution, which was at least about 50 µL (micro liters). In view of the genome medical treatment in the future, it is desirable that the amount of the sample taken from the patient for analyzing the sample by use of the array, i.e., the sample containing the target, to be small.

### Disclosure of Invention

A first object of the present invention, which has been achieved in view of the situation described above, is to provide a method of detecting one of two components collectively forming a specific coupling pair consisting of a probe and a target, the component providing the probe being spotted and immobilized into solid phase on a substrate surface, and the other component being detected as the target, and is intended to improve the efficiency of the specific coupling reaction between the two components, to lower the background noise and to decrease the amount of the sample solution used. Further, the present invention is intended to realize a reaction having a high reliability in the detecting method described above.

A second object of the present invention is to provide an apparatus for working the detecting method described above.

Further, a third object of the present invention is to provide an article useful for the detecting method described above.

For achieving the first object in the present invention, a solution containing a target substance is regioselectively disposed on the prove region in which trace amount of probe is immobilized on, for example, a micro-array substrate by using, for example, a spotting device.

To be more specific, the first object of the present invention is achieved by providing a method of carrying out a specific coupling reaction between first and second components collectively forming a specific coupling pair, which method comprises:
supplying the second component onto a plurality of isolated reaction regions formed on the substrate surface and having the first component arranged therein in advance,
wherein a liquid material containing the second component is regioselectively supplied in a manner to superpose the first component arranged on the reaction region, in accordance with the address of each of the reaction regions.

The second object of the present invention is achieved by providing an apparatus for detecting a target substance within a large number of isolated reaction regions on a substrate surface by using at least two kinds of the reacting components, the apparatus comprising:
a holding means for holding the substrate having a large number of isolated reaction regions formed on the surface, each of the reaction regions having the first component immobilized thereon;
an address storing means for storing the address of the each reaction region on the substrate surface;
a second component supply means for regioselectively supplying a liquid material containing the second component onto the reaction region of the substrate surface so that the liquid material is within a region substantially equal in size to each of the reaction regions; and
a control means for controlling the second component supply means based on the address of each of the plural reaction regions so as to permit the liquid material containing the second component to be regioselectively arranged on the first component within each of the reaction regions.

Further, the third object of the present invention is achieved by providing an article that is prepared to perform the detecting reaction of a target substance within a plurality of isolated reaction regions on a substrate surface by using first and second components collectively forming a specific coupling pair and at least one kind of a third reaction component;
wherein at least two of the first, second and third reaction components, which are lacking at least one reaction components required for the detecting reaction, are regioselectively superposed one upon the other within the reaction region under the state that these components can be stored.

### Brief Description of Drawings

FIG. 1 shows a conventional method of detecting a gene by using a micro-array;
FIG. 2 shows a method of detecting a gene by using a micro-array according to one embodiment of the present invention;
FIG. 3 is a flowchart showing the steps of the method shown in FIG. 2;
FIG. 4 shows a first modification of the detecting method according to the embodiment of the present invention shown in FIG. 2;
FIG. 5 shows a second modification of the detecting method according to the embodiment of the present invention shown in FIG. 2;
FIG. 6 shows a third modification of the detecting method according to the embodiment of the present invention shown in FIG. 2;
FIG. 7 shows a fourth modification of the detecting method according to the embodiment of the present invention shown in FIG. 2;
FIG. 8 is a block diagram showing the construction of the gist portion of the apparatus according to the present invention;
FIG. 9 is an perspective view exemplifying the construction of a substrate used in the present invention;
FIGS. 10A and 10B collectively show a method of arranging a liquid material containing a second component on a first component immobilized in advance on the substrate surface shown in FIG. 9 by using an apparatus of the present invention; and
FIGS. 11A, 11B and 11C collectively show a method of arranging a liquid material containing a second component on a first component immobilized in advance on the substrate surface shown in FIG. 9 by using an apparatus of the present invention.

### Best Mode for Carrying Out the Invention

The present invention will now be described in detail in respect of each category.

### <Invention of Method>

The term "substrate" in the present invention denotes a solid phase member having a plurality of isolated regions in which a biological material such as a nucleic acid, protein or a cell can be arranged and immobilized on the same plane. The surface of the substrate is sufficient for forming a plurality of two-dimensional or three-dimensional regions in which the biological material can be arranged. It is desirable to use, for example, a micro-array comprising a platelike substrate such as a slide glass, a plastic plate, a silicon wafer or a porous filter and a biological material that is regioselectively arranged in a plurality of isolated regions on the surface of the substrate. The micro-array typically includes a substrate in which the portion for arranging the biological material has a two-dimensional plane. Alternatively, it is also possible for the substrate to include a three-dimensional recess or a hollow portion (see Japanese Patent Announcement (Kohyo) No. 9-504864). Further, a one-dimensional micro-array, i.e., a linear micro-array, is also known to the art (see WO 01/051207).

In the present invention, use is made of first and second components collectively forming a specific coupling pair. The pair of components represents, for example, a combination of a probe substance and a target substance. Examples of the combinations of the first and second components used in the present invention include, but not limited to, a combination of a nucleic acid such as a gene and a complementary strand thereof, a combination of an antigen and an antibody, a combination of an allergen and an antibody, and a combination of an enzyme and a substrate. It is possible for a detectable label such as a fluorescent dye, a chemical luminescent substance, a magnetic substance or a gold colloid to be coupled with one or both of these first and second components. These biological materials can be used in the form of a liquid phase or a solid phase as far as the volume of the biological material is small enough to be arranged in a plurality of relatively small isolated regions on the substrate surface. Also, where a protein cell that is a membrane component, such as an antigen, an allergen or a receptor is used as the first component or the second component, it is possible to use the protein, which is extracted by lysis treatment of the cell, in the form of a liquid phase or a solid phase. It is also possible to use the cell itself as it is. There is a known protein array in which the extracted protein is arranged in a plurality of isolated regions on the substrate surface (see WO 00/04382). In addition, also known is a cell array in which the cell itself is arranged in place of the extracted protein (see WO 00/17624). Where a nucleic acid is used as the first component or the second component, it is possible to use any of DNA, RNA or a modified nucleic acid such as PNA as the nucleic acid. These nucleic acids are designed to have a desired length, base sequence and conformation (single chain, double chain, cyclic, linear), and be used in the form of a liquid phase or a solid phase having the nucleic acid content adjusted at an appropriate level. Further, the first or second component can be in the form of a solid phase reagent such as beads, which has been immobilized with the first or second component by coating the beads with a liquid phase biological material or by wetting the beads with the liquid phase biological material.

In the present invention, it is possible to use at least one kind of a third component as required in addition to the first and second components described above. The third component used in the present invention includes, for example, a component promoting or initiating the specific coupling reaction between the first component and the second component, a nucleic acid primer, DNA polymerase, ligase, exonuclease, and a multivalent antibody (secondary antibody). In the competitive reaction, the third component includes, for example, a ligand, an inhibitor or a membrane protein digesting agent (protease), which are common to the first component and the second component.

The term "reaction region" in the present invention denotes the region on the substrate in which a specific coupling reaction is carried out between the first component and the second component. It is possible for the reaction region to be a region defined in advance by forming, for example, a recessed section. However, the reaction region need not be such a predefined region. In this case, the reaction region is defined by the first component arranged in advance.

In the method of the present invention, the staying region of the target solution can be limited to a region in the vicinity of the probe by regioselectively supplying a solution containing the second component (e.g., a target substance) onto the first component (e.g., a probe) in a superposing fashion, i.e., in a manner to cover the first component. Also, since it is possible for the target solution to obtain a spatial thickness, the diffusion effect of the target solution can be expected so as to increase the specific coupling efficiency of the target substance that is supplied in a superposing fashion onto the probe.

In the present invention, the presence of the target solution in any region other than the region in which the probe is arranged in advance is eliminated, with the result that the back ground noise coming from other than the reaction region is considered to be eliminated.

In the present invention, the target solution is regioselectively supplied onto the probe in a superposing fashion, i.e., in a manner to cover the probe, by using the technology of ink jetting or mechanical spotting and, thus, only several nanoliters (nL) of the target solution is required for a single spot, i.e., single reaction region. As a result, the required amount of target solution is decreased in the step of the hybridization reaction so as to save the target solution.

As described above, the target solution can be saved so as to achieve cost reduction.

Incidentally, it is particularly effective in the present invention to supply regioselectively a sample onto the region in which the probe is immobilized to a solid phase in a superposing fashion, i.e., in a manner to cover the particular reaction region noted above. It is also possible to supply onto the particular region in a superposing fashion various reagents required for the analysis in place of the sample. It follows that the technical scope of the present invention also covers the case where various reagents are supplied in a covering fashion onto the region in which the probe is immobilized to a solid phase.

With reference to FIGS. 2 and 3, we will describe in the following, the detecting method according to a first embodiment of the present invention in which a nucleic acid such as gene is detected by using a micro-array in which a nucleic acid probe is immobilized. FIG. 2 illustrates the detecting method of the first embodiment, and FIG. 3 is a flowchart showing the steps for carrying out the method of the first embodiment.

In this embodiment, a trace amount of a solution 7 containing a target is supplied in a superposing fashion onto a trace amount of a probe 6 such as DNA immobilized on a micro-array substrate 5. A spotting device such as a pin-type device or an ink jet arrayer is used for supplying the solution 7.

In the first step, the micro-array substrate 5 having the probe immobilized thereon is mounted on a spotting device such as an ink jet arrayer. In this step, it is desirable to use a slide glass having a position-aligning index-mark put on, for example, an edge of the substrate having the probe immobilized thereon, in order to permit the solution 7 containing the target to be supplied onto the immobilized probe 6 in a superposing fashion, i.e., in a manner to cover the immobilized probe 6. After the index-mark is aligned, the target solution 7 is supplied onto the immobilized probe 6 in a manner to superpose thereon so as to permit the target to be spotted on the immobilized probe 6. Further, in order to prevent the evaporation of the target solution during the hybridization, the probes and the target-containing solution that are arranged spot-like are covered with a lid member 8.

According to the method described above, a trace amount of probes such as DNA, which are immobilized on the micro-array substrate, form a hybrid with the target labeled with the fluorescent molecule within the target-containing solution. After the hybridization reaction, the expression frequency of the target gene can be measured by measuring the amount of fluorescent light emitted from the target hybridized with the probe which has been immobilized on the micro-array in advance.

According to the present invention, the technology of ink jetting and mechanical micro-spotting, which was used in prior arts for immobilizing the probe, can also be applied to the hybridization test. According to this embodiment of the present invention, the target is supplied in a superposing fashion onto a trace amount of the probe immobilized on the micro-array substrate so as to permit the target to be positioned closer to the probe, leading to an improved efficiency of the hybridization. Also, since the hybridization solution does not permeate into a region other than the probe, the background noise is lowered so as to facilitate the analysis. Further, in the conventional system in which the target solution was allowed to permeate into a free space between the cover glass and the slide glass, it was necessary to use a large amount of target solution, i.e., 50 µL of target solution. In this embodiment of the present invention, however, it suffices to use only about 1 nL (nanoliter) of target solution for a single spot so as to markedly save the amount of target solution.

Incidentally, each constituent element of the first embodiment can be variously modified or changed naturally. For example, in the first embodiment, a slide glass is used as the substrate. Alternatively, it is also possible to use substrates of various materials such as a silicon wafer, as well as various shapes and sizes. However, since a fluorescent dye is often contained in the target solution, it is desirable to use, for example, the substrate made of a glass that does not emit fluorescent light. This is because such the substrate enables to decrease the background noise from the substrate in analyzing the micro-array.

Modifications of the first embodiment described above will now be described.

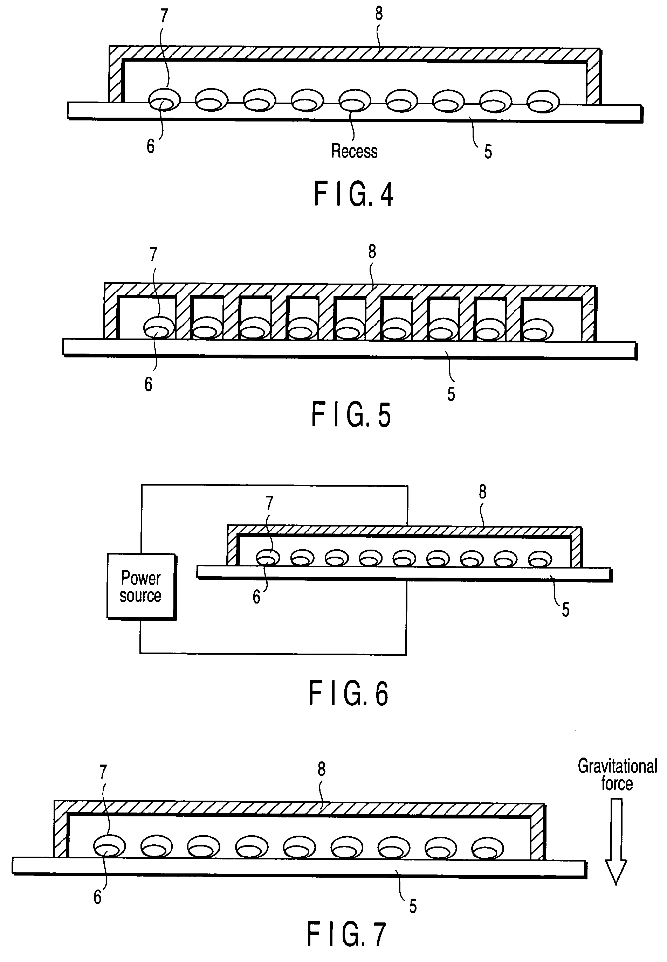

### Modification 1 (FIG. 4):

In this modification, the isolated reaction regions on the substrate are slightly recessed in order to permit the target to be positioned closer to the probe when a target solution is supplied onto the reaction regions having the probe immobilized thereon in a superposing fashion, i.e., in a manner to cover the probe.

### Modification 2 (FIG. 5):

The cover substrate has a capillary structure so as to permit the cover substrate to be partitioned for each spot.

### Modification 3 (FIG. 6):

Where the probe and the target used are substances having an electric charge such as DNA, an electric charge is applied from the lower portion of the substrate. As a result, the probe and the target are attracted toward the substrate so as to carry out the hybridization effectively.

### Modification 4 (FIG. 7):

Gravitational force is applied in carrying out the hybridization reaction.

### <Invention of Apparatus>

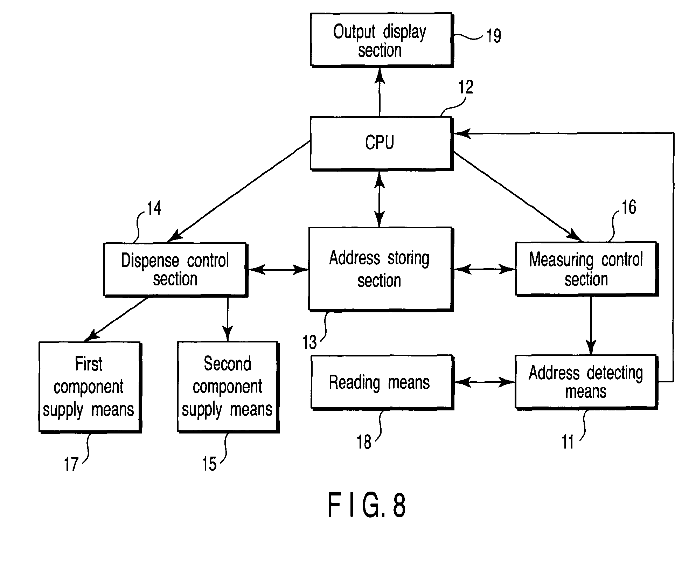

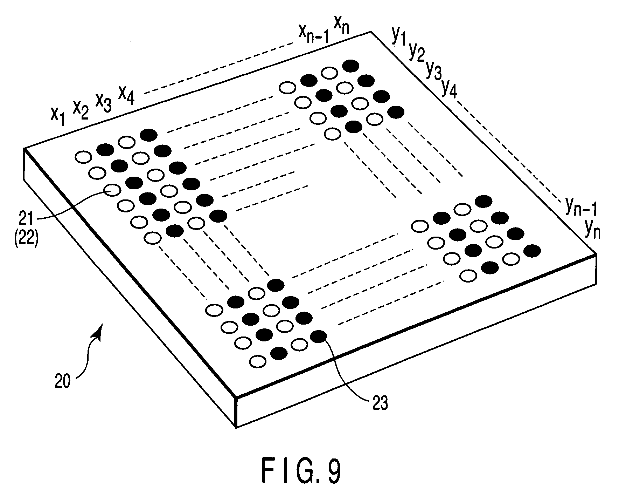

The present invention is also directed to an apparatus for carrying out the method of the present invention described above. An apparatus according to one embodiment of the present invention will now be described with reference to FIGS. 8 and 9.

FIG. 8 is a block diagram showing the construction of the gist portion of the apparatus of the present invention. As shown in the drawing, the apparatus of the present invention comprises an address detecting means 11, a central processing unit (CPU) 12, an address storing section 13, a dispensing control section 14, a reagent (second component) dispenser 15, a measuring section 16, an output display section 19, a reading means 18, and a sample dispenser 17. Signals are transmitted between these members of the apparatus as denoted by arrows in the drawing.

The apparatus also comprises a holding means (not shown) for holding a substrate 20 having a plurality of isolated reaction regions 21 formed on the surface as shown in FIG. 9. The reaction regions 21 are arranged to form a matrix array on the surface of the substrate 20. The address of each reaction region 21 is designated by the combination (xₙ, yₙ) of the position on the X-axis and the position on the Y-axis, the X- and Y-axes collectively forming a coordinate. Also, a first component 22 required for carrying out a prescribed reaction is arranged in advance on each reaction region 21, and a second component 23 denoted by a black dot is arranged on the first component 22. The second component 23 is shown in only some of the reaction regions 21 in FIG. 9. However, it is possible to arrange the first component 22 and the second component 23 in all the reaction regions 21. It is also possible for the kinds of first component 22 arranged in these large number of reaction regions 21 to be equal to or to differ from each other. Likewise, it is possible for the kinds of second component 23 arranged in these large number of reaction regions 21 to be equal to or to differ from each other. It is also possible to arrange, for example, the same kind of first component in the reaction regions forming the same row or the same column. Likewise, it is possible to arrange the same kind of first and second component in the reaction regions forming the same row or the same column. Further, it is possible to arrange a different kind of first and second component in the reaction regions forming a different row or a different column.

The address detecting device 11 included in the apparatus of the present invention serves to detect the address of each reaction region 21 (the address of the first component 22) on the surface of the substrate 20 held by the holding means described above. The detected address is stored in the address storing section 13. The central processing unit (CPU) 12 transmits an instruction to the dispensing control section 14 in accordance with the address information so as to control the reagent dispenser 15 for arranging the second component. As a result, the second component is regioselectively dispensed onto the first component 22 in a superposing fashion, i.e., in a manner to cover the first component. Incidentally, the first component supply means 17 for arranging the first component is also included in the apparatus so as to make it possible to carry out the operation starting with the arrangement of the first component 22 by using the substrate 20 not having the first component arranged thereon in advance. The first component supply means 17 is also controlled by the dispensing control section 14. The first component and the second component as well as an appropriate third component, as required, are arranged one upon the other in a superposed fashion. After completion of a prescribed detecting reaction, the CPU 12 transmits an electrical signal to the measuring control section 16 so as to detect the result of the prescribed detecting reaction via the detecting means 11. To be more specific, the detecting means 11 detects a detectable label such as a fluorescent marker in respect of each reaction region 21, and the result of the detection is transmitted to the CPU 12. The detected signal is subjected to arithmetic processing in the CPU 12, and the CPU 12 permits the result of the arithmetic processing to be displayed on the appropriate output display section 19 such as a CRT monitor.

Each member of the apparatus shown in FIG. 8 will now be described in detail.

The principle of the address detection by the detecting device 11 is to recognize the difference in the characteristics in terms of materials science between the substrate 20 and the first component 22 arranged in the reaction region 21 on the substrate 20. Such being the situation, it is desirable for the address detecting device 11 to be a physical means, preferably to have an optical measuring function or another measuring function that can be associated with the optical measuring data. Desirably, the sites where the first component is present and is not present are detected on the basis of the difference in the transmittance and/or the reflectance of wave energy (light or ultrasonic wave) so as to recognize the presence of the first component 22. The recognition signal is subjected to arithmetic processing in the CPU 12 so as to determine the address of the first component 22 (i.e., the address of the reaction region 21) on the substrate surface. It follows that it is desirable for the address detecting device 11 to further include means for converting the detection signal given from the substrate 20 into an electrical signal adapted for the arithmetic processing carried out by the CPU 12.

Where, for example, the light transmittance of the substrate 20 is larger than that of the first component 22, the detection data obtained from the address detecting device 11 is adapted for forming an image of the arrangement layout of the substrate 20 and all the sites of the first component 22 on the substrate 20. On the other hand, where the difference in the light transmittance is detected by a light beam, provided are a shadow image or a color image of the substrate 20 and the first component 22.

Where the detection is performed on the basis of the difference in light reflectance, it is possible to convert the thickness distribution on the substrate into image data by covering the substrate surface with a light-reflective material (e.g., silicon oxide) and using a surface plasmon resonance method or a polarization measuring means such as an eripsometer. Conversely, where the first component contains a light scattering polymer or a metal colloid, it is possible to form an image as an optical shadow image. It is possible to improve the detection accuracy by measuring both the light transmittance and the light reflectance.

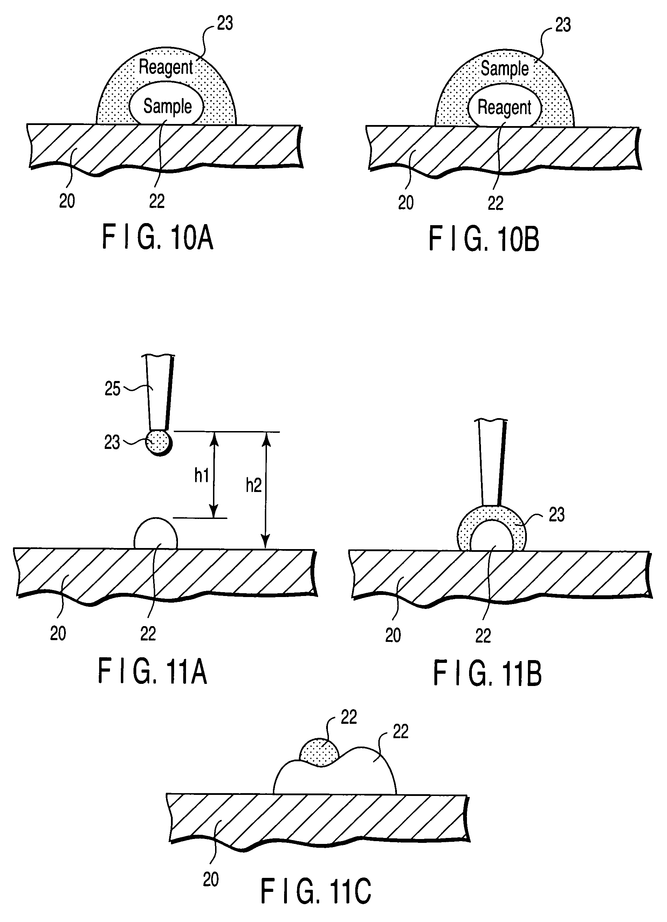

The distribution image of the first component 22 on the substrate 20 reflects accurately not only the individual address of the first component 22 but also the two-dimensional or three-dimensional volume of the first component 22. Preferably, the address detecting means includes the reading means 18 for reading the two-dimensional or three-dimensional shape and/or size of the first component 22 on the basis of the lengths on the x-, y- and z-axes of the first component 22. The reading means 18 determines the center of the first component 22 as the address information. It follows that, in dispensing the succeeding second component, it is possible to dispense the second component toward the center noted above. As a result, a liquid material containing the second component 23 can be regioselectively supplied onto the first component 22 arranged in advance on the substrate 20 in a manner to cover the first component 22, as shown in FIG. 10A. It is shown in the drawing that the first component 22a is a sample such as a cell, and the second component 23 is a reagent such as an antigen. However, it is also possible for the first component 22 is a reagent such as a DNA probe and the second component 23 is a sample containing DNA such as a gene.

Where the first component 22 is sized relatively large entity such as a cell, it is possible to select a specified portion of the first component 22 so as to determine the center of the specified portion. In this case, it is advantageous to utilize an imaging means such as a CCD camera or a laser scanning device in order to detect a shadow image of light or a color image for forming an image. If the beam diameter of the light beam in the laser scanning device is made slightly larger than the volume of the first component, it is possible to increase the imaging speed. On the other hand, if the beam diameter is made smaller than the volume of the first component, it is possible to obtain the detailed information within the volume of the first component. Since the cell used as the first component 22 contains in general many kinds of valuable reaction factors on the surface or inside thereof, it is advantageous to obtain detailed information from the first component 22. It follows that, where the detecting means includes a confocal optical system, it is possible to know not only the layout of the first component 22 on the substrate 20 but also the optical characteristics for every specified portion within the first component 22. In the case of arranging the every second component that carries out a specific reaction for every specified portion of the first component, it suffices to arrange the every second component in a manner to cover selectively the specified portion of the first component. Also, in the case of applying a reagent like protease that permits dissolving only partially the cell membrane, it suffices to arrange the second component in a manner to cover the dissolved portion alone on the upper surface of the cell used as the first component 22.

It is advantageous to design the apparatus such that the address detecting means 11 can be similarly utilized in the subsequent step of obtaining the result of the biological reaction carried out for the first component in each address after addition of all the required components including the second component.

Incidentally, the address detecting means 11 is indispensable in the case where the first component is arranged in advance on the substrate 20. In other words, it is not absolutely necessary to obtain the address information by the address detecting means 11 in the case where the operation is started with the arrangement of the first component, i.e., the case where the first component and the second component are arranged simultaneously or sequentially. It should be noted in this connection that, in the particular case noted above, it is possible to obtain an effective address information from the dispensing control section 14 for controlling the movement of the dispenser apparatus in the directions of the x-, y- and z-axes, said dispenser apparatus acting as an arranging means for arranging the first component. It follows that it is not absolutely necessary to obtain the address information by the address detecting means 11 in the case where the operation is started with the arrangement of the first component, as pointed out above.

It is possible for the address storing section 13 to be formed of any memory means such as a random access memory (RAM) capable of storing the address information of the first component 22 transmitted from the address detecting means 11 or from the reading means 18 and also capable of reading the stored information as required.

The second component supply means 15 is formed of a dispenser equipped with a spurting nozzle for regioselectively arranging a liquid material containing the second component in a superposing fashion on the first component 22 arranged in advance on the substrate 20. The second component supply means 15 is controlled by the dispensing control section 14 so as to have the position of the spurting nozzle determined in accordance with the address information of the first component 22 stored in the address storing section 13. To be more specific, the position of a nozzle 25 of the dispenser is determined such that the nozzle 25 is positioned right above the first component 22 as shown in FIG. 11A. In this case, the height of the nozzle 25 is controlled by the dispensing control section 14 so as to obtain a desirable supply height of the second component 23 in accordance with the shape of the substrate 20 or the first component 22. For example, where the substrate has recesses or fine holes, it is advisable for the tip of the nozzle 25 to be positioned at a relatively small height (h₁) in accordance with the height of the arrangement of the first component 22 on the substrate 20. Conversely, where the first component 22 having a certain height is arranged on the surface of the substrate 20, it is desirable for the tip of the nozzle 25 to be positioned at a height (h₂) equal to the sum of the height (h₁) noted above and an appropriate height within the upper limit of the height of the first component 22. Where the substrate 20 is not permeable and the first component 22 is in a liquid phase or includes a very thin layer (e.g., a thickness not larger than 1 µm), it is possible for the tip of the nozzle 25 to be positioned at an appropriate close distance calculated from the upper surface of the substrate 20. The contact means such as the stamp device can be driven by the contact force proportional to the height noted above.

A liquid material containing the second component 23 is supplied under the state described above so as to form a liquid droplet containing the second component 23 on the tip of the nozzle 25. Then, the nozzle 25 is moved downward toward the substrate 20 so as to bring the droplet of the liquid material containing the second component 23 into contact with the first component 22. As a result, the second component 23 is spread in a manner to cover the entire region of the first component 22 as shown in FIG. 11B. Where the second component 23 is to be arranged in a manner to cover the entire region of the first component 22 as described above, the nozzle 25 is moved downward such that the tip of the nozzle 25 faces the center of the first component 22. However, where the first component 22 is formed of a cell and it is intended to arrange locally the second component 23 in the recessed portion of the cell as shown in FIG. 11C, the tip of the nozzle 25 is moved downward toward the recessed portion of the cell in accordance with the information obtained by the reading means 18.

Incidentally, the total supply amount of the second component 23 is determined in accordance with the volume of the first component 22. In this case, the second component supply means 15 supplies an excessively large amount of the second component 23 that is slightly larger than the required total amount and, then, the excess portion of the second component 23 is sucked out immediately. The particular method is desirable in that the supply error caused by the viscosity or the surface tension of the second component 23 can be eliminated.

Also, where the first component 22 is substantially dried (e.g., a humidity of 60% or less) or where the first component 22 includes a solid phase portion, it is possible to supply the second component 23 at a relatively high supply rate. However, where the first component 22 is under a wet state (e.g., humidity of 70% or more) or substantially forms a liquid phase (e.g., a humidity of 90% or more), it is desirable to supply the second component 23 at a supply rate at which the first component 22 is not broken by the shearing or perforation. It should be noted that a control signal conforming to the first component 22 is generated in advance from the dispensing control section 14 so as to control the driving of the pressure generating device (e.g., pump, syringe, or piezo element) included in the second component supply means 15, thereby controlling the supply rate of the second component 23.

Further, in the description given above, a single dot of the arranged first component 22 occupies the entire region of the reaction region (spot) 21. However, it is possible for a plurality of isolated dots of the first component 22 to be formed in the single reaction region (spot) 21 (see Japanese Patent Disclosure No. 2002-65274). In other words, it is possible for n x n dots of the first component 22 formed by the ink jetting to be present in a single spot 21. In this case, the second component 23 can be supplied in a manner to cover each dot of the first component 22 under the state that the arranged area of the second component 23 is large enough to cover each dot of the first component 22. The second component 23 can be dispensed like the first component 22 as a dot-like liquid droplet having a diameter slightly larger than that of the dot of the first component 22 by using an ink jet dispenser. The ink jet dispenser permits controlling the number of dots at the same number even in the case of using different devices. In the case of supplying the second component 23 in the form of liquid droplets each having a diameter larger than that of the dot of the first component 22 in a manner to superpose the dot of the first component 22, the number of said liquid droplets being equal to the number of dots of the first component 22, it is possible to cover all the dots of the first component 22 within a single reaction region (spot) 21 with the liquid droplets at the lowest cost even if the mechanical error is taken into account. If necessary, it is possible to monitor the amount and/or state of the dot-like liquid droplets containing the second component 23 by using the detecting device described above so as to navigate the operation of supplying the second component 23 in a manner to superpose the first component 22. Also, where a set of a plurality of finely divided dots of the first component 22 are present within the reaction region (spot) 21, it is possible to spot the second component 23 in the central portion of the set in the form of a dot-like liquid droplet having a diameter 1.1 to 1.5 times as large as that of the set of the finely divided dots of the first component 22. It is possible for 10,000 to 10,000,000 finely divided dots of the first component 22 to be present in a single reaction region (spot) 21, and it suffices for 10 to 500 dots of the second components 23 to be present in a single reaction region (spot) 21. Within the same reaction region (spot) 21, it is possible for a plurality of liquid droplets of the second component 23 to be connected partially or entire to each other so as to form a continuous liquid phase. It is possible to form a continuous liquid phase in which a plurality of dots of the second component 23 are connected to each other by repeatedly spurting the second component 23 by using an ink jet type dispenser in the form of dots, each dot having a diameter equal to or smaller than that of the dot of the first component 22. As a result, the entire region of the first component 22 is covered with the second component 23. As far as the region within a single reaction region (spot) is concerned, a plurality of connected liquid droplets of the second component 23 do not bring about any disadvantage in respect of the reaction and the measurement. On the other hand, where finely divided dots of the first component 22, which are not covered with the second component 23, are present, even if a few numbers, within the reaction region (spot) 21, it is possible for a noise derived from the nonspecific reaction to be brought about. According to statistical probability, the first component 22 and the second component 23 may overlap each other with a high probability in the case where the first component 22 and the second component 23 are spurted onto the same reaction region of the substrate 20 under the state that the first component 22 and the second component 23 are exactly equal to each other in the dot diameter and the number of dots. However, the present invention provides an arrangement that permits decreasing the amount of the first component 22 that is not covered with the second component 23 as much as possible (preferably, comply eliminating) in order to improve the statistical reliability and to improve the S/N ratio. In the case of using a porous substrate provided with pores having a diameter of 1 to 50 µm, a plurality of dots of the second component 23 arranged within each reaction region (spot) 21 are connected to each other so as to form a continuous liquid phase, thereby producing the effect that each spot 21 is filled completely with the liquid phase of the second component 23. On the other hand, where the each spot 21 is not filled completely with the continuous liquid phase, it is possible for noise caused by the bubble or dust to be generated in the reaction step or the measuring step. In addition, it is possible in the present invention to arrange a biologically inactive third component on the entire surface of the substrate 20 in a manner to cover the first component 22 and the second component 23 arranged on the substrate 20. It is possible for the third component to be an aqueous liquid such as a buffer solution or purified water, or an oily liquid such as silicone oil, an olive oil or liquid paraffin. The oily third component produces the effect of preventing the evaporation of the first component and the second component in the reacting step and the measuring step. Use of a curable transparent resin as the third component is advantageous not only in the effect of preventing the evaporation but also in the transfer of the substrate inside and outside the apparatus.

The first component supply means 17 is a dispenser similar to the second component supply means 15. The dispenser constituting the first component supply means 17 is used in the case where the first component 22 is not arranged in advance on the substrate 20 and, thus, in the case where the operation is started with the arrangement of the first component 22 for carrying out a desired detecting reaction. In an integral system for supplying the liquid materials of both the first component and the second component, it is desirable for both supply means 15 and 17 to be mounted to a common nozzle-moving mechanism. Where the nozzle-moving mechanisms are used separately, the moving history of the first component supply means 17 is stored in the address storing section 13 in conjunction with the address information of the substrate 20. The second component supply means 15 gains access to each address of the substrate 20 in accordance with the moving history of the first component supply means 17, which is read from the address storing section 13, so as to supply the second component 23 onto the first component 22 without fail. Where the second component 23 is supplied onto the substrate 20 having the first component 22 arranged thereon in advance, it is unnecessary to use the first component supply means 17.

Where the arrangement of the first component 22 and the arrangement of the second component 23 are performed in different places by using different devices, it is desirable to mount on a part of the substrate 20 a coded information storing means (e.g., a bar code or a magnetic recording medium) relating to the address of the first component 22 arranged on the substrate 20, as an information transmitting system between the first component supply means 17 and the second component supply means 15. To be more specific, an appropriate coding means (e.g., a barcode printer or a magnetic recording apparatus) is allowed to perform its function such that a code, in which the moving history of the first component supply means 17 for arranging the first component 22 is related to the address information of the substrate 20, is imparted to the coded information storing means. Then, prior to the regioselective supply of the second component 23 onto the first component 22, the coded information storing means on the substrate is read by the reading means 18, and the decoded address information is stored in the address storing section 13. Further, the dispensing control section 14 is allowed to drive the second component supply means 15 based on the information given from the address storing section 13. If such an information transmitting system is used as a cooperating means, it is possible to supply the second component 23 onto the first component 22 in a manner to superpose the first component 22 without requiring the address detection of the first component 22 by the address detecting means 11 even in the case where the first component supply means 17 and the second component supply means 15 are independent separate devices. Also, the particular information transmitting system permits preventing an erroneous recognition of the individual substrates in an apparatus which handles a large number of substrates.

### <Invention of Article (Micro-array)>

According to the method and apparatus of the present invention described above, it is possible to provide an article that is prepared to carry out a detecting reaction of a target substance within a plurality of isolated reaction regions on the substrate surface, by using first and second components forming a specific coupling pair and at least one kind of a third reaction component, i.e., an article, which can be stored and which is prepared in advance such that a desired reaction can be performed by arranging, for example, a final component.

To be more specific, the article of the present invention, e.g., a micro-array, is characterized in that at least two of the first, second and third reaction components, which are a plurality of components failing to satisfy all the reaction components required for the detecting reaction, are regioselectively arranged in a superposed fashion within the reaction regions, and that these superposed plural components are in a storable state.

An example of the micro-array obtained by applying the technical idea of the present invention has a construction equivalent to that of the substrate 20 shown in FIG. 9. In each of the reaction regions 21, the first component 22 and the second component 23 are regioselectively arranged in an superposed fashion as shown in FIGS. 10A and 10B. In the micro-array according to the present invention, however, a reaction is not carried out by using the first component 22 and the second component 23 alone, and a desired reaction is carried out only when the third component is added. Combinations of the first component, the second component and the third component include the examples given below:

### Example 1:

First component: cell;
Second component: primer, substrate base, DNA polymerase;
Third component: protease;

### Example 2:

First component: HCV antigen;
Second component: anti-immuno globulin antibody marked with fluorescent light;
Third component: anti-HCV antibody;

In order to make it possible to store the first component 22 and the second component 23 covering the first component 22 in the micro-array, these components can be subjected to freeze drying in the present invention so as to put these components under a dried state. In this case, it is desirable for a denaturation preventing agent such as a saccharide to be present together with the first and second components in order to prevent the protein from being denatured during the storage. The saccharides used for this purpose include, for example, sucrose, treharose, glucose, dextrin and xylose. Also, in order to make it possible to store the first component 22 and the second component 23, it is possible to cover the substrate 20 with a shielding means such as oil materials (e.g., silicone oil, olive oil and fluid paraffin) so as to shield the first and second components from the ambient air.

As described above in detail, the present invention makes it possible to obtain prominent effects in detecting a target substance by using a probe immobilized into solid phase on the substrate surface. For example, it is possible to improve the efficiency of the specific coupling reaction between the probe and the target substance, to suppress background noise and to decrease the amount of sample liquid used.

## Claims

1. A method of carrying out a specific coupling reaction between first and second components collectively forming a specific coupling pair, which method comprises:
supplying the second component onto a plurality of isolated reaction regions formed on the substrate surface and having the first component arranged therein in advance,
wherein a liquid material containing the second component is regioselectively supplied in a manner to superpose the first component arranged on the reaction region, in accordance with the address of each of the reaction regions.

2. The method according to claim 1, **characterized in that** the first component is formed of at least one cell.

3. The method according to claim 1 or 2, **characterized in that** the first component and the second component are formed of substances including a nucleic acid such as a gene, an antigen, an antibody, an allergen, a hormone and an enzyme.

4. The method according to claim 1, **characterized in that** the substrate surface has a plurality of convex portions and concave portions, each of the reaction regions on which the first component is immobilized is formed in each of the concave portions.

5. An apparatus for detecting a target substance within a large number of isolated reaction regions on a substrate surface by using at least two kinds of the reacting components, said apparatus comprising:
a holding means for holding the substrate having a large number of isolated reaction regions formed on the surface, each of said reaction regions having the first component immobilized thereon;
an address storing means for storing the address of the each reaction region on the substrate surface;
a second component supply means for regioselectively supplying a liquid material containing the second component onto the reaction region of the substrate surface so that the liquid material is within a region substantially equal in size to each of the reaction regions; and
a control means for controlling the second component supply means based on the address of each of the plural reaction regions so as to permit the liquid material containing the second component to be regioselectively arranged on the first component within each of the reaction regions.

6. The apparatus according to claim 5, wherein the control means controls the second component supply means such that the back-and-forth movement of the second component supply means relative to the substrate and the supply rate of a liquid material containing the second component by the supply means are changed in accordance with the kind of the first component and/or the second component and/or the amount of the liquid material containing the second component.

7. The apparatus according to claim 5, wherein the control means controls the second component supply means such that the back-and-forth movement of the second component supply means relative to the substrate and the supply rate of the liquid material are changed in accordance with the arranged state of the first component and the arranged state of the liquid material containing the second component.

8. The apparatus according to claim 5, **characterized by** further comprising a first component supply means for supplying a liquid material containing the first component, wherein the supplying operation of the first component supply means and the second component supply means is cooperated with the same or different substrates.

9. The apparatus according to claim 5, **characterized by** further comprising a detecting means for detecting the arranged state of the liquid material on the substrate, wherein the relative positions between the second component supply means and the substrate or the first component are adjusted in accordance with the result of the detection by the detecting means.

10. The apparatus according to claim 5,
**characterized by** further comprising a reading means for reading the shape or the kind of the first component, wherein the supplying operation of the second component supply means is changed in accordance with the result of the reading by the reading means.

11. An article that is prepared to perform the detecting reaction of a target substance within a plurality of isolated reaction regions on a substrate surface by using first and second components collectively forming a specific coupling pair and at least one kind of a third reaction component;
**characterized in that** at least two of the first, second and third reaction components, which are lacking at least one reaction components required for the detecting reaction, are regioselectively superposed one upon the other within the reaction region under the state that these components can be stored.

12. The article according to claim 11, **characterized in that** the plural superposed components are freeze-dried under the state that a anti-freeze-dry preserving agent is present together with the superposed components.

13. The article according to claim 11, **characterized in that** the plural superposed components are shielded from the ambient air by a shielding means.

14. The article according to claim 13, **characterized in that** the component in the uppermost layer of the superposed plural components is in the form of an aqueous solution, and the shielding means is formed of an oil layer covering the aqueous solution.
